# EUROPEAN PATENT APPLICATION

(11) **EP 4 775 663 A1**
(43) Date of publication of application: **15.07.2026**
(21) Application number: 25224264.9
(22) Date of filing: 17.12.2025
(51) Int. Cl.: C12N 1/20, C12R 1/125

(54) **METHOD AND APPLICATION FOR SELECTING FUNCTIONAL MICROBIOME IN LOCAL SOIL**

(30) Priority: 17.12.2024 US 202463735019 P
(71) Applicant: Feng Yu Biogreen Tech Co. Ltd., Xizhi Dist New Taipei City 221432 (TW)
(72) Inventor: CHANG, Winston, 221432 New Taipei City (TW); CHENG, Tzu-Wei, 221432 New Taipei City (TW); WU, Ri-Gui, 221432 New Taipei City (TW)
(74) Representative: Wittmann, Günther

(57) **Abstract**

The present invention relates to a method for selecting functional microbiome and its applications. The method comprises: providing a local soil sample; subjecting the local soil sample to a pretreatment process and filtration to obtain a first microbial suspension; culturing the first microbial suspension using a culture formulation to obtain a second microbial suspension; and performing plate identification. Subsequently, the microbiome is subjected to microbial domestication, and the domesticated microbiome is introduced into the local soil to achieve the effect of soil restoration.

## Description

### CROSS-REFERENCE TO RELATED APPLICATIONS

This application claims priority to and the benefit of U.S. Provisional Application No. 63/735,019, filed on December 17, 2024, the disclosure of which is incorporated by reference in its entirety.

### BACKGROUND OF THE INVENTION

### 1. FIELD OF THE INVENTION

The present invention relates to systems and methods for selecting composite microbial populations for soil restoration. More particularly, the present invention relates to selecting and domestication of functional microbiome derived from local soil, which possess soil restoration capabilities, and applying such functional microbiome to the restoration of the corresponding local soil.

### 2. DESCRIPTION OF THE PRIOR ART

Soil restoration refers to remediation and recovery measures performed on an imbalanced soil environment to address various conditions present within the soil. These conditions may arise from naturally occurring environmental factors or anthropogenic activities. Exemplary environmental factors include permafrost in cold regions that hinders crop cultivation, or excessive soil salinity that prevents vegetation from surviving. Anthropogenic factors include, for example, industrial waste discharged into the environment, excessive use of chemical fertilizers and pesticides, and the release of nuclear waste.

Methods for soil restoration include bioremediation and physicochemical treatments, depending on the characteristics of the region and the type of contamination. Bioremediation primarily utilizes microorganisms to degrade or absorb toxic substances. For example, certain bacteria can degrade hydrocarbons following petroleum spills. Microorganisms may also be employed to remediate contaminants in soil by introducing pollutant-degrading microbial strains into contaminated sites, where metabolic processes convert pollutants into harmless substances. Additionally, beneficial microorganisms may be introduced to release advantageous compounds in the soil, thereby increasing organic matter content and enhancing soil fertility. Compared with conventional chemical treatment methods, bioremediation offers advantages such as lower cost and environmental friendliness, and has therefore garnered increasing attention and application.

However, current bioremediation approaches for soil restoration frequently rely on introducing a single beneficial microbial strain into the environment. In practical applications, such strains often fail to adapt to the local conditions, resulting in poor bioremediation performance or requiring an extended period before adaptation occurs. Accordingly, developing an effective bioremediation method that can be applied across diverse soil conditions and significantly reduce the time required for soil restoration is an important challenge of considerable interest in the field.

### SUMMARY OF THE INVENTION

In one aspect, the present invention provides a method for selecting functional microbiome, comprising:
providing a local soil sample;
performing a pretreatment on the local soil sample to obtain a pretreated soil sample, and
filtering the pretreated soil sample to obtain a first microbial suspension;
culturing the first microbial suspension using a culture formulation to obtain a second microbial suspension; and
plating the second microbial suspension to determine the content of *Bacillus subtilis* therein, wherein the second microbial suspension is designated as functional microbiome when the content of *Bacillus subtilis* exceeds 5% of the total microbial population.

In some embodiments, the local soil sample comprises earthworms and/or metabolites derived from earthworms.

In some embodiments, the pretreatment comprises a heat treatment and/or an extractant treatment.

In some embodiments, the heat treatment comprises heating the local soil sample in an extractant at a temperature of 60-90°C for 30-60 minutes.

In some embodiments, the extractant treatment comprises adding an extractant to the local soil sample to perform extraction.

In some embodiments, the extractant comprises at least one selected from the group consisting of water, physiological saline, culture medium, a bacitracin-containing culture medium, a monoculture supernatant, a saline suspension, and a metal ion suspension.

In some embodiments, the culture formulation comprises at least one selected from the group consisting of glucose, glycerol, molasses, starch, arabinose, fructose, galactose, lactose, maltose, mannose, sucrose, peanut meal, soybean meal, corn gluten meal, beef extract, peptone, yeast powder, fish meal, urea, spent fungal biomass, fermented residue with ammonium salts, nitrate salts, ammonia water, yeast extract, sodium chloride, magnesium chloride, sodium bicarbonate, monopotassium phosphate, dipotassium phosphate, ammonium sulfate, ammonium chloride, sodium citrate, sodium malate, sodium carbonate, and potassium carbonate.

In another aspect, the present invention provides a method for soil restoration, comprising:
providing the functional microbiome selected according to the method of claim 1;
subjecting the functional microbiome to microbial domestication to obtain domesticated functional microbiome, wherein the microbial domestication comprises culturing the functional microbiome in a bioreactor and regulating microenvironmental parameters in the bioreactor to simulate the environment of the local soil sample; and
introducing the domesticated functional microbiome into the local soil.

In some embodiments, the microenvironmental parameters comprise nutrient composition, nutrient concentration, pH, temperature, humidity, mechanical force, and a growth medium.

In some embodiments, the growth medium comprises wood chips, rice husks, and soil.

Conventional bioremediation approaches for soil restoration typically involve selecting a single microbial strain that is beneficial to soil health and introducing the selected strain into the soil for restoration. In the present invention, however, variations in soil environments across different regions-such as differences in humidity, temperature, pH, and native microbial communities-often prevent a single microbial strain from adapting to diverse environmental conditions, thereby limiting the effectiveness of restoration.

In the present invention, the complete native microbial community is directly obtained from the soil targeted for restoration. Undesirable microorganisms are removed through pretreatment, and the remaining microbial population is cultivated using a specially formulated culture formulation. The cultivation results are verified through plating identification, thereby enabling the selection of functional microbiome. The selected functional microbiome are subsequently introduced back into the target soil environment to achieve concrete benefits, such as environmental remediation or enhancement of soil health.

The functional microbiome described in the present invention may undergo microbial domestication prior to being introduced into the target soil environment. The functional microbiome are cultured in a bioreactor, and the parameters of the bioreactor are adjusted to simulate natural environmental conditions, thereby facilitating domestication of the functional microbiome into a microbial community adapted to the target environment. The domesticated functional microbiome are then introduced into the soil region requiring restoration. This domestication process can significantly shorten the time needed for environmental restoration.

### BRIEF DESCRIPTION OF THE DRAWINGS

FIG. 1 is a flowchart illustrating the method for selecting functional microbiome disclosed in the present invention.
FIG. 2 is a flowchart illustrating the soil restoration method disclosed in the present invention.
FIG. 3 is an image showing the plating results obtained after pretreatment under different heating temperatures and durations.
FIG. 4 is an image showing the plating identification results of different microbial samples on a selective culture medium.
FIG. 5 is a comparison image illustrating seed germination results after three days of cultivation in local soil (bitter gourd cultivation soil) following the introduction of composite microbiome, functional microbiome, and a single microbial strain.
FIG. 6A is a photograph of a bitter gourd (*Momordica charantia*) cultivation field prior to applying the functional microbiome disclosed in the present invention. FIG. 6B is a photograph of the bitter gourd (*Momordica charantia*) cultivation field after applying the functional microbiome disclosed in the present invention.
FIG. 7 is a comparison image illustrating seed germination results after three days of cultivation in local soil (*Camellia oleifera* cultivation soil) following the introduction of composite microbiome, functional microbiome, and a single microbial strain.
FIG. 8A is a photograph of a *Camellia oleifera* cultivation field prior to applying the functional microbiome disclosed in the present invention. FIG. 8B is a photograph of the *Camellia oleifera* cultivation field after applying the functional microbiome disclosed in the present invention.
FIG. 9A is a photograph of a *Brassica rapa chinensis* cultivation field prior to applying the functional microbiome disclosed in the present invention. FIG. 9B is a photograph of the *Brassica rapa chinensis* cultivation field after applying the functional microbiome disclosed in the present invention.

### DETAILED DESCRIPTION OF THE PREFERRED EMBODIMENT

Additional technical aspects, features, and advantages of the present invention will become apparent from the following detailed description of preferred embodiments in conjunction with the accompanying drawings.

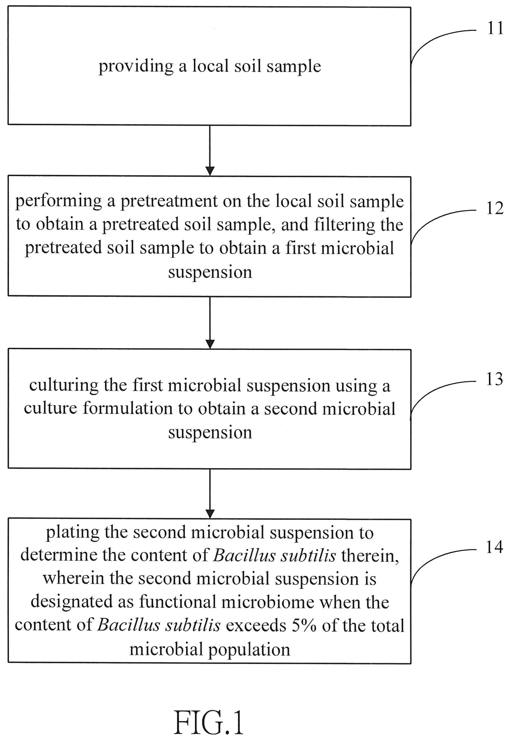

In one aspect, the present invention provides a method for selecting functional microbiome. Referring to FIG. 1, the method comprises steps 11 to 14.

Step 11: providing a local soil sample.

In some embodiments, the local soil sample contains earthworms and/or metabolites derived from earthworms. In some embodiments, the local soil sample comprises native microbial populations characteristic of the region of origin.

Step 12: performing a pretreatment on the local soil sample to obtain a pretreated soil sample, and filtering the pretreated soil sample to obtain a first microbial suspension.

In some embodiments, the pretreatment comprises a heat treatment and/or an extractant treatment. The pretreatment may be performed using one or more physical or chemical procedures. In the present invention, the sequence in which the pretreatment steps are performed is not limited. For example, the heat treatment may be performed prior to the extractant treatment, or the extractant treatment may be performed prior to the heat treatment.

In some embodiments, the heat treatment is performed by adding the local soil sample into an extractant and heating the mixture. The temperature of the heat treatment ranges from 60°C to 90°C, for example, 60°C, 65°C, 70°C, 75°C, 80°C, 85°C, or 90°C. The heating duration ranges from 30 to 60 minutes, for example, 30 minutes, 35 minutes, 40 minutes, 45 minutes, 50 minutes, 55 minutes, or 60 minutes.

In some embodiments, the extractant treatment comprises adding an extractant to the local soil sample to perform extraction.

In some embodiments, the extractant is a liquid capable of solubilizing microorganisms and comprises at least one selected from the group consisting of water, physiological saline, a general culture medium, a bacitracin-containing culture medium, a monoculture supernatant, a saline solution, and a metal ion solution.

In some embodiments, the bacitracin-containing culture medium is not limited to the use of bacitracin. Bacitracin functions to inhibit undesirable microorganisms so as to increase the relative proportion of beneficial microbial strains. Any antimicrobial agent known to those skilled in the art may be used in place of bacitracin. The monoculture supernatant may suppress the growth of other microorganisms to facilitate the growth of the monoculture strain. In some embodiments, the monoculture strain is *Bacillus subtilis.* In addition, substances present in the supernatant (postbiotics) may further promote the growth of various symbiotic probiotics.

In some embodiments, the filtering is performed by centrifugation filtration or paper filtration, the primary purpose of which is to remove interference from solid components in the sample.

Step 13: culturing the first microbial suspension using a culture formulation to obtain a second microbial suspension.

In some embodiments, the culture formulation comprises at least one selected from the group consisting of glucose, glycerol, molasses, starch, arabinose, fructose, galactose, lactose, maltose, mannose, sucrose, peanut meal, soybean meal, corn gluten meal, beef extract, peptone, yeast powder, fish meal, urea, spent fungal biomass, fermented residue with ammonium salts, nitrate salts, ammonia water, yeast extract, sodium chloride, magnesium chloride, sodium bicarbonate, monopotassium phosphate, dipotassium phosphate, ammonium sulfate, ammonium chloride, sodium citrate, sodium malate, sodium carbonate, and potassium carbonate. In some embodiments, the culture formulation contains elements such as carbon, hydrogen, oxygen, nitrogen, phosphorus, sulfur, potassium, calcium, magnesium, boron, manganese, zinc, molybdenum, cobalt, iodine, and copper.

In some embodiments, the culture formulation comprises culture materials that promote the growth of *Bacillus subtilis.*

In some embodiments, different local soil samples require different culture formulations. For example, slightly acidic or slightly alkaline soil conditions may require adjustment of the pH of the culture formulation. Moreover, different local soils contain different trace elements (such as metal ions). Therefore, the culture formulation in the present invention may be adjusted according to the specific characteristics of the local soil environment.

Step 14: plating the second microbial suspension to determine the content of *Bacillus subtilis* therein, wherein the second microbial suspension is designated as functional microbiome when the content of *Bacillus subtilis* exceeds 5% of the total microbial population.

In some embodiments, the content of *Bacillus subtilis* accounts for 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 45%, 50%, 55%, 60%, 65%, 70%, 75%, 80%, 85%, or 90% or more of the total microbial population.

More specifically, the identification in the present invention is performed by plating the cultivated microbiome to determine the microbial composition. By observing the growth characteristics of *Bacillus subtilis,* the proportion of *Bacillus subtilis* within the microbiome can be confirmed. When *Bacillus subtilis* constitutes at least 5% of the total colony composition, the microbiome is identified as the functional microbiome in the present invention and can be subsequently applied to soil restoration. In the present invention the functional microbiome is for soil restoration.

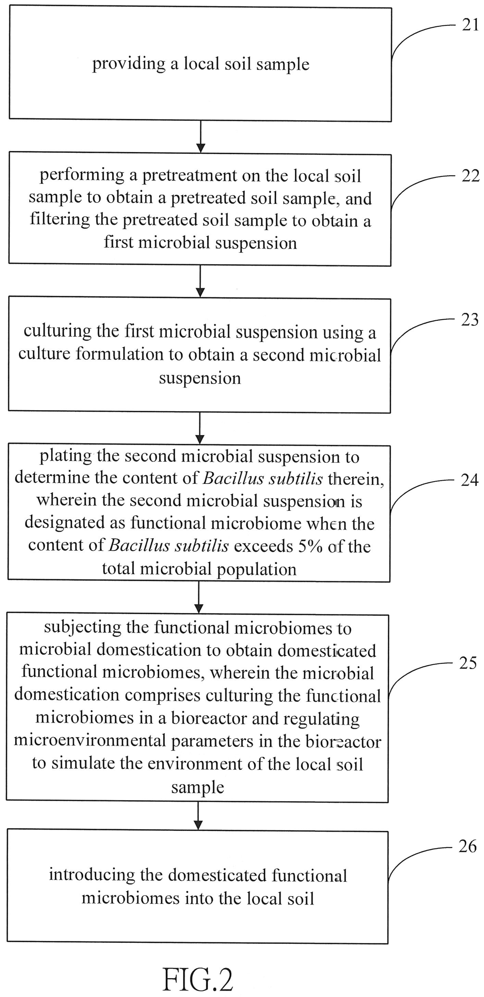

In another aspect, the present invention provides a soil restoration method. Referring to FIG. 2, the method comprises steps 21 to 26.

In some embodiments, the functional microbiome selected using the selecting method disclosed in the present invention is subjected to microbial domestication. The microbial domestication comprises cultivating the functional microbiome in a bioreactor and regulating the microenvironmental parameters within the bioreactor to simulate the environment of the local soil sample, thereby enabling the functional microbiome to better adapt to the local soil environment during iterative cultivation and scale-up. The microenvironmental parameters include, for example, nutrient composition, nutrient concentration, pH, temperature, humidity, external forces, growth medium, and the content of other microbial species. The growth medium includes solid and liquid growth media such as wood chips, rice husks, soil, and other porous materials.

In some embodiments, the functional microbiome that has undergone microbial domestication may be further subjected to immobilization. The carrier used for immobilization includes, but is not limited to, wood chips or calcium carbonate. The immobilization process comprises placing a solid carrier into a mixing apparatus, activating the mixing device, and then adding the microbial suspension. The mixing ratio ranges from 1:2 to 1:4. After thorough mixing, the mixture is dried and subsequently stored.

In some embodiments, the immobilized functional microbiome may be present in various forms, such as solid powders, granules, or solid particles.

In some embodiments, the soil restoration method in the present invention comprises introducing the domesticated functional microbiome into the local soil, for example, by broadcasting, irrigation, or mixing into the environment. In some embodiments, the domesticated functional microbiome is introduced into the imbalanced soil environment together with nutrient components, wherein the nutrient components serve as nutrient sources for microbial growth. For example, the nutrient components may include organic waste derived from food-processing or agricultural and livestock operations, which are commonly used in compost applications. In some embodiments, the functional microbiome selected and domesticated in the present invention is most suitable for application in the region from which the microbial population originates. However, the present invention is not limited to introducing the functional microbiome only into its region of origin; the functional microbiome may also be introduced into regions exhibiting similar environmental problems to achieve environmental restoration.

It should be understood that the above general description and the detailed description below are both exemplary and explanatory, and are not intended to limit the present invention. Certain details of one or more embodiments of the present invention are described in the following disclosure. Other features or advantages of the present invention will be apparent from the non-exhaustive list of representative embodiments below and from the accompanying claims.

Unless otherwise defined herein, scientific and technical terms used in connection with the present disclosure shall have the meanings that are commonly understood by those of ordinary skill in the art. The terminology used in this specification is used to describe particular embodiments only and is not intended to limit the invention.

As used herein, the singular form "a", "an", and "the" includes plural references unless indicated otherwise. For example, "an" excipient includes one or more excipients. The term "or" is used interchangeably with the term "and/or" unless the context clearly indicates otherwise.

As used interchangeably herein, "around", "about" and "approximately" shall generally mean plus or minus 1% of the numerical value of the number with which it is being used. Numerical quantities given herein are approximate, meaning that the term "around", "about" or "approximately" can be inferred if not expressly stated.

The phrase "comprising" as used herein is open-ended, indicating that such embodiments may include additional elements. In contrast, the phrase "consisting of" is closed, indicating that such embodiments do not include additional elements (except for trace impurities). The phrase "consisting essentially of" is partially closed, indicating that such embodiments may further comprise elements that do not materially change the basic characteristics of such embodiments.

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well-known in the art. Generally, nomenclatures used in connection with, and techniques of biochemistry, enzyme science, molecular and cell biology, microbiology, immunology, medicine, pharmacy, cell culture and microbial cultivation described herein are those well-known and commonly used in the art. The methods and techniques of the present disclosure are generally performed according to conventional methods well known in the art and as described in various general and more specific references that are cited and discussed throughout the present specification unless otherwise indicated.

As used herein, the term "functional microbiome" generally refers to a microbial population having soil restoration capability, which is obtained through cultivation and selection according to the method disclosed in the present invention. It should be understood that the functional microbiome does not pertain to a single microbial strain, but rather to a collection of different microbial species that has been characterized and confirmed to meet specific criteria. In the present invention, the specific criterion is defined as *Bacillus subtilis* constituting more than 5% of the total microbial population. Only microbial populations meeting this requirement fall within the definition of "functional microbiome." As used herein, the term "composite microbiome" refers to a microbial population obtained through cultivation using the method disclosed in the present invention. More specifically, the composite microbiome is the microbial population resulting from the pretreatment and cultivation with the culture formulation described in the present invention. If subsequent identification confirms that the content of *Bacillus subtilis* exceeds 5% of the total microbial population, the composite microbiome may be designated as the "functional microbiome."

As used herein, the term "local soil sample" refers to a sample obtained from the soil of a given region, with the primary purpose of acquiring the native microbial populations specific to that region. Because the sample originates from the region of interest, the microorganisms therein inherently possess the ability to adapt to the environmental conditions of that region. For example, a local soil sample may be obtained directly from the soil of the region or from the root zone of plants growing in the region. Such samples can provide the native microbial populations characteristic of the local environment. Sampling may also be performed within earthworms, which likewise yields native microbial populations specific to the region. As used herein, the term "local soil" refers to soil obtained directly from the geographic region in which the functional microbiome is intended to be applied for soil restoration. The local soil contains the native microbial populations, environmental characteristics, and physicochemical properties specific to that region. Because the soil sample originates from the target region, the microorganisms present therein inherently exhibit adaptability to the environmental conditions of that region, including but not limited to humidity, temperature, pH, nutrient composition, and naturally occurring microbial communities.

As used herein, the term "microbial domestication" refers to culturing microorganisms in an environment that is not their natural habitat, such that the microbial community grows into a stable state that meets human-designated expectations. The non-natural environment includes, but is not limited to, a bioreactor.

As used herein, the term "microenvironmental parameters" refers to the numerical indicators representing environmental conditions under natural settings, including but not limited to organic matter composition, nutrient composition, nutrient concentration, pH, temperature, humidity, growth media, original microbial species and/or their abundance, as well as solid or liquid growth media such as wood chips, rice husks, soil, and other porous materials.

As used herein, the term "soil fertility" refers to the ability of soil to support plant growth and development, particularly by providing essential nutrients, water, gases, and adequate physical structure to promote healthy root system development. Higher soil fertility indicates that the soil is more suitable for the growth of crops and/or plants.

As used herein, the term "soil restoration" refers to the process of recovering soil ecosystems that have been damaged due to human activities, natural disasters, and/or climatic factors, restoring the soil to a state close to its natural or original condition, or even improving it beyond its original condition. Examples include increasing biodiversity and enhancing organic matter content.

The present invention is further illustrated by the following embodiments, which are not to be construed as limiting the scope of the present invention in any manner. Unless otherwise specified, the experimental methods used in the following embodiments are conventional methods. Unless otherwise indicated, the materials, reagents, instruments, and equipment used in the embodiments may be obtained commercially.

### Example 1 Soil Restoration in a Bitter Gourd Cultivation Field

In the present example, a local soil sample was obtained from the natural environment. Using the method for selecting the functional microbiome disclosed in the present invention, beneficial microorganisms present in the local soil were selected and subsequently subjected to scale-up cultivation. The cultivated functional microbiome was then introduced back into the local soil to observe changes in soil fertility.

In the present embodiment, the local soil sample was collected from an infertile bitter gourd (*Momordica charantia*) cultivation field located in Xinshe District, Taichung City (Taiwan). The soil sample was subjected to heat treatment and filtration, followed by cultivation using the culture formulation disclosed in the present invention. The microbial composition was examined by plating, and samples in which the *Bacillus subtilis* content exceeded 5% of the total microbial population were identified as the functional microbiome for this embodiment. The functional microbiome was subsequently scaled up and applied to the infertile cultivation field.

### Selection of the Functional Microbiome

### 1. Pretreatment of Soil Samples

A 10 g soil sample was added into an Erlenmeyer flask containing 100 ml of physiological saline (0.85% NaCl + ddH₂O). The flask opening was sealed with aluminum foil, and the mixture was subjected to heat treatment in water baths at 60°C, 70°C, or 90°C for 30 minutes or 60 minutes. After heat treatment, the sample was filtered via gravity filtration by placing a funnel on an Erlenmeyer flask and positioning a folded filter paper within the funnel. The heat-treated sample was gently poured along the edge of the filter paper until 50 ml of filtrate had been collected. The filtration apparatus was then removed, and the collected filtrate served as the first microbial suspension in the present invention.

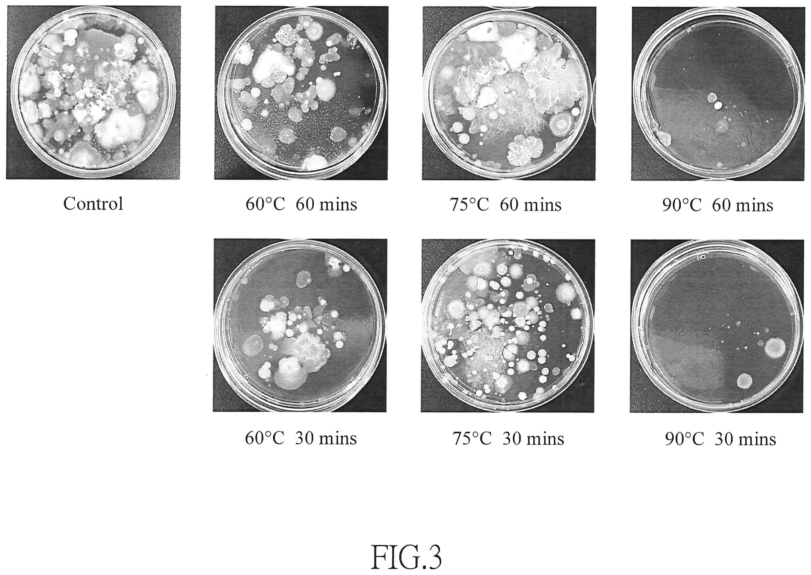

The first microbial suspensions obtained under each heat treatment condition (60°C for 60 minutes, 70°C for 60 minutes, 90°C for 60 minutes, 60°C for 30 minutes, 70°C for 30 minutes, and 90°C for 30 minutes) were subjected to plating identification. The control group consisted of samples that were not heat-treated. The results, shown in FIG. 3, indicate that different temperatures and durations of heat treatment resulted in significant changes in microbial composition. Different local soil environments are suitable for different combinations of temperature and heating duration. In the present embodiment, most contaminating microorganisms in the samples were eliminated under the 90°C heat treatment, resulting in a relatively simplified microbial community for subsequent cultivation.

### 2. Microbial Cultivation

Using a pipette, 1 ml of the first microbial suspension was transferred into 100 ml of the culture formulation. The mixture was incubated in a shaking incubator at 30°C and 150 rpm for 24 hours to obtain the second microbial suspension in the present invention. The culture formulation comprised at least one selected from the group consisting of glucose, glycerol, molasses, starch, arabinose, fructose, galactose, lactose, maltose, mannose, sucrose, peanut meal, soybean meal, corn gluten meal, beef extract, peptone, yeast powder, fish meal, urea, spent fungal biomass, fermented residue with ammonium salts, nitrate salts, ammonia water, yeast extract, sodium chloride, magnesium chloride, sodium bicarbonate, monopotassium phosphate, dipotassium phosphate, ammonium sulfate, ammonium chloride, sodium citrate, sodium malate, sodium carbonate, and potassium carbonate.

In the present example, the culture formulation was an acidic culture medium comprising 1-4 g/L of an inorganic nitrogen source selected from ammonium sulfate, ammonium nitrate, ammonium chloride, or combinations thereof; 10-25 g/L of a phosphate buffer system comprising K₂HPO₄ and KH₂PO₄; 0.5-2 g/L of an organic acid salt selected from sodium citrate, sodium malate, or combinations thereof; 0.05-0.5 g/L of magnesium ions; and 3-10 g/L of a carbon source selected from glucose, sucrose, fructose, molasses, or combinations thereof.

### 3. Plating Identification

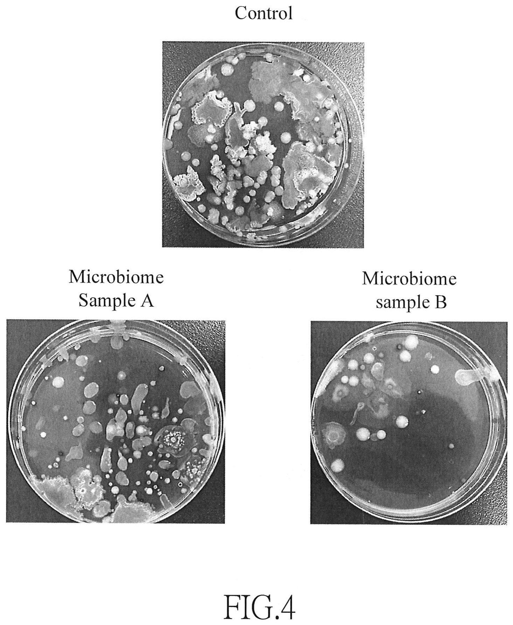

The second microbial suspensions obtained under various cultivation conditions were subjected to plating identification. The plating medium used was the HIMEDIA Bacillus selective medium (HiCrome Bacillus Agar Base, purchased from HIMEDIA). The plates were incubated at room temperature for one day. The control group consisted of native microbial populations that did not undergo the microbial cultivation process. The plating results are shown in FIG. 4. In the present embodiment, an image analysis system was further employed to quantify the color distribution and relative abundance of colonies on each plate. Different microbial species produced colonies of different colors, among which green colonies represented *Bacillus subtilis.* The calculated results are shown in Table 1.

In the control group, *Bacillus subtilis* accounted for only 1.02% of the total colony composition. In contrast, *Bacillus subtilis* accounted for 9.18% of the total colony composition in microbiome sample A after cultivation, thereby meeting the criterion disclosed in the present invention requiring the proportion of *Bacillus subtilis* to exceed 5%. Accordingly, microbiome sample A was identified as the functional microbiome and was used in subsequent experiments and for local soil restoration.

In comparison, *Bacillus subtilis* accounted for only 3.82% of the total colony composition in microbiome sample B after cultivation, which does not satisfy the >5% criterion defined in the selecting method of the present invention. Therefore, microbiome sample B is not considered the functional microbiome in the present invention and is referred to merely as a composite microbiome.

**Table 1. Analysis of Bacillus subtilis from various bacterial samples**

| Samples | *Bacillus subtilis* | | |
|---|---|---|---|
| | Colony composition (%) | Percentage of whole dish (%) | Area (cm²) |
| Control | 1.02 | 0.47 | 0.32 |
| Microbiome sample A (functional microbiome) | 9.18 | 5.02 | 3.19 |
| Microbiome sample B (composite microbiome) | 3.82 | 2.00 | 1.27 |

### Seed Germination Test

In the present example, 38 g of local soil was placed into each culture dish. Subsequently, 2 g of the functional microbiome disclosed in the present invention, the composite microbiome (i.e., the microbial sample in which the *Bacillus subtilis* content did not exceed 5%), or a single microbial strain (*Bacillus subtilis*) was added to the corresponding culture dishes. Fifteen bok choy (*Brassica rapa* var. *chinensis*) seeds were then placed into each dish. After three days of cultivation, the seed germination rate of each treatment group was examined. The control group consisted of local soil without the addition of any microbial strains.

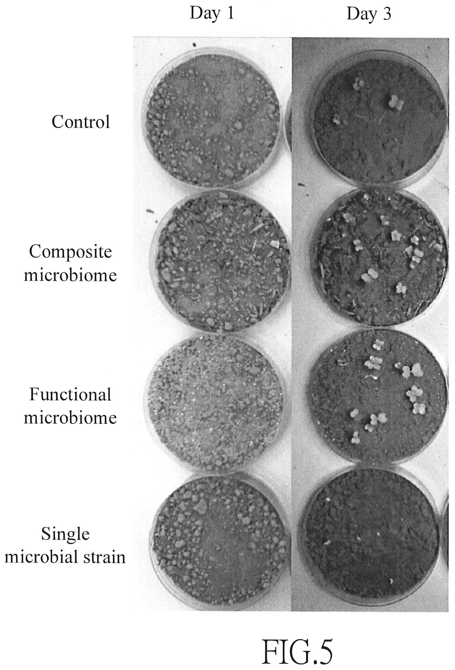

The experimental results are shown in Table 2 and FIG. 5. After three days of cultivation, only 3 seeds germinated in the control group, corresponding to a germination rate of approximately 20%, indicating that the original local soil was highly infertile. In contrast, the group treated with the functional microbiome disclosed in the present invention exhibited 11 germinated seeds, corresponding to a germination rate of approximately 73%, which was superior to both the control group and the groups treated with the composite microbiome or the single microbial strain. These results demonstrate that the functional microbiome selected using the method of the present invention effectively enhances seed germination when reintroduced into the local soil and exhibits soil restoration capability.

**Table 2. Germination rate test of soil in bitter gourd cultivation field**

| | Control | Composite microbiome | Functional microbiome | Single microbial strain |
|---|---|---|---|---|
| Number of germinated seeds (unit) | 3 | 9 | 11 | 1 |
| Germination rate (%) | 20% | 60% | 73% | 6.7% |

### Restoration of Local Soil

In the present example, the functional microbiome (microbiome sample A) selected as described above was cultivated in a bioreactor, and the microenvironmental parameters within the bioreactor were regulated to simulate the environment of the local soil sample. The microenvironmental parameters included nutrient composition, nutrient concentration, pH, temperature, humidity, external forces, and growth media. These conditions allowed the functional microbiome to undergo microbial domestication and scale-up cultivation.

In the present example, the microbial domestication conditions included adjusting the pH to 5.5-6.5, controlling the temperature between 28-35°C, maintaining a shaking frequency of 80-120 rpm, controlling the moisture content of the culture medium at 40-70% (w/w), and maintaining a relative humidity of 40-80%. The cultivation was carried out for approximately 48-72 hours.

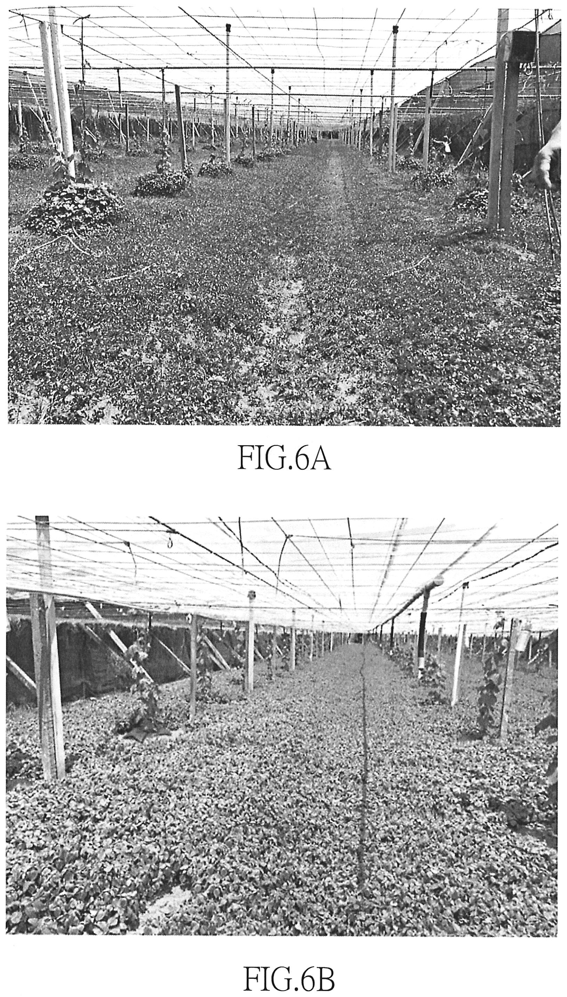

In the present example, the domesticated functional microbiome was subsequently applied to the infertile cultivation field. As shown in FIG. 6A, prior to the application of the functional microbiome selected using the method disclosed in the present invention, the soil in the cultivation field was severely infertile and plant growth was poor. However, as shown in FIG. 6B, three months after applying the functional microbiome selected by the method of the present invention, the soil fertility in the region was significantly improved, and plant growth conditions were markedly enhanced. These results demonstrate that the functional microbiome selected according to the present invention possesses soil restoration capability.

### Example 2 Soil Restoration in a Camellia oleifera Cultivation Field

In the present embodiment, a local soil sample was collected from an infertile *Camellia oleifera* cultivation field located in Jiadong Village, Hsinchu City (Taiwan). The local soil sample contained earthworms and earthworm metabolites. The soil sample was subjected to pretreatment and filtration, followed by cultivation using the culture formulation disclosed in the present invention. The microbial composition was examined by plating, and samples in which the *Bacillus subtilis* content exceeded 5% were identified as the functional microbiome for this embodiment. The selected functional microbiome was subsequently subjected to microbial domestication, scale-up cultivation, and application to the infertile cultivation field. The pretreatment, cultivation, selection, and microbial domestication procedures used in the present embodiment are as described in Example 1.

In the present example, the culture formulation was a neutral culture medium comprising 3-10 g/L of protein hydrolysates selected from tryptone, soybean protein hydrolysate, or combinations thereof; 1-5 g/L of yeast extract; 0-5 g/L of beef extract; 3-10 g/L of sodium chloride; and 0-5 g/L of a carbon source selected from glucose, molasses, glucose syrup, or combinations thereof.

### Seed Germination Test

In the present embodiment, 38 g of local soil was placed into each culture dish. Subsequently, 2 g of the functional microbiome disclosed in the present invention, the composite microbiome (i.e., the microbial sample in which the *Bacillus subtilis* content did not exceed 5%), or a single microbial strain (*Bacillus subtilis*) was added to the corresponding culture dishes. Fifteen bok choy (*Brassica rapa* var. *chinensis*) seeds were then placed into each dish. After three days of cultivation, the seed germination rate of each treatment group was evaluated. The control group consisted of local soil without the addition of any microbial strains.

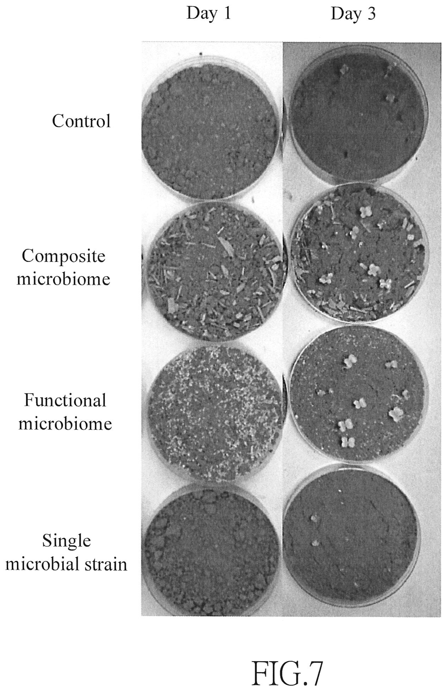

The experimental results are shown in Table 3 and FIG. 7. After three days of cultivation, only 4 seeds germinated in the control group, corresponding to a germination rate of approximately 27%, indicating that the original local soil was severely infertile. In contrast, the group treated with the functional microbiome disclosed in the present invention exhibited 11 germinated seeds, corresponding to a germination rate of approximately 73%. This result was superior to the control group as well as the groups treated with the composite microbiome or the single microbial strain. These findings demonstrate that the functional microbiome selected using the method disclosed in the present invention effectively improves seed germination when reintroduced into the local soil and exhibits soil restoration capability.

**Table 3. Germination rate test of soil in Camellia oleifera cultivation field**

| | Control | Composite microbiome | Functional microbiome | Single microbial strain |
|---|---|---|---|---|
| Number of germinated seeds (unit) | 4 | 8 | 11 | 1 |
| Germination rate (%) | 27% | 53% | 73% | 6.7% |

### Restoration of Local Soil

In the present example, the functional microbiome selected according to the present invention was subjected to microbial domestication and scale-up cultivation, followed by application to the local soil. In the present embodiment, the microbial domestication conditions included adjusting the pH to 6.8-7.6, maintaining the temperature between 30-37°C, operating at a shaking frequency of 80-120 rpm, supplying an aeration rate of 0.5-2 vvm in the fermenter, controlling the moisture content of the culture medium at 40-65% (w/w), and maintaining a relative humidity of 40-70%. The cultivation was carried out for approximately 24-48 hours.

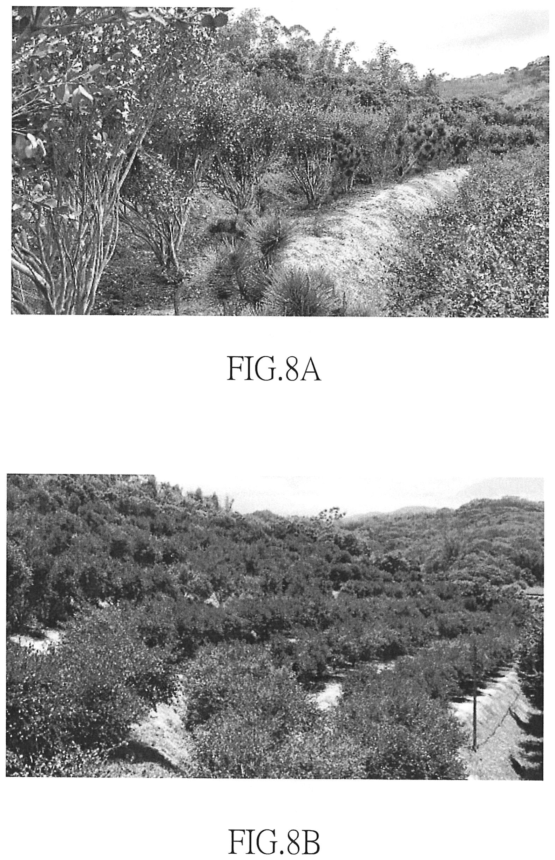

As shown in FIG. 8A, prior to the application of the functional microbiome selected using the method disclosed in the present invention, the cultivation field exhibited severely infertile soil, lacked groundcover vegetation, and produced only sparse flowers and fruits. However, as shown in FIG. 8B, four months after applying the functional microbiome selected according to the present invention, the soil fertility in the region significantly improved; groundcover vegetation restored vitality; plant growth became vigorous; and flower and fruit production increased substantially. These observations demonstrate that the functional microbiome selected using the method disclosed in the present invention possesses soil restoration capability.

### Example 3 Soil Restoration in a Brassica rapa chinensis Cultivation Field

In the present example, a local soil sample was collected from a *Brassica rapa chinensis* cultivation field affected by high salinity, located in Erlun Township, Yunlin County (Taiwan). The collected soil sample was subjected to pretreatment and filtration, followed by cultivation using the culture formulation disclosed in the present invention. The cultivated sample was plated to determine its microbial composition, and samples in which the *Bacillus subtilis* content exceeded 5% were identified as the functional microbiome for this embodiment.

The selected functional microbiome was subsequently domesticated in a bioreactor, during which the microenvironmental parameters were adjusted to simulate the soil conditions under high-salinity stress. After 3 to 5 days of microbial domestication, the functional microbiome was immobilized and stored. When needed, the immobilized microbiome was subjected to scale-up cultivation and applied to the high-salinity cultivation field to evaluate improvements in soil EC value and soil compaction. The pretreatment, cultivation, selection, and microbial domestication procedures used in the present example are as described in Example 1.

In the present example, the culture formulation was an alkaline medium comprising 3-10 g/L of protein hydrolysates; 1-10 g/L of yeast extract; 0.5-3 g/L of phosphate; 0.05-0.5 g/L of magnesium ions; 5-15 g/L of a carbon source selected from glucose, starch, maltose, molasses, or combinations thereof; and 2-10 g/L of alkaline salts selected from sodium carbonate, sodium bicarbonate, potassium carbonate, or combinations thereof.

In the present example, the microbial domestication conditions included adjusting the pH to 8.5-10.5, maintaining the temperature at 30-42°C, operating at a shaking frequency of 100-150 rpm, controlling the moisture content of the culture medium at 35-65% (w/w), and maintaining a relative humidity of 40-70%. The cultivation was performed for approximately 4-48 hours.

In this example, solid-state preservation of the microbiome was performed by placing wood chips in an autoclave for sterilization at 121°C for 30 minutes, followed by drying at 60°C. The wood chips served as the solid carrier. The microbial suspension and the wood chips were mixed uniformly at a ratio of 1:2 (w/w), and the mixture was subsequently stored in a drying oven at 45°C.

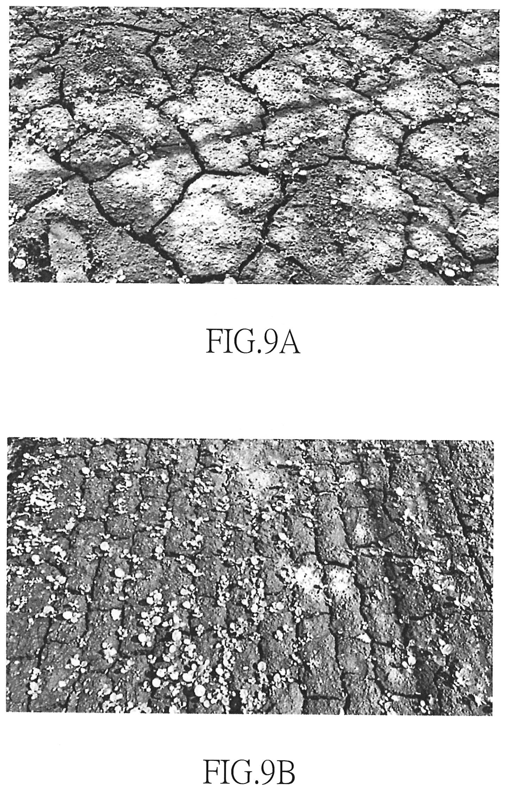

As shown in FIG. 9A, prior to applying the functional microbiome selected using the method disclosed in the present invention, the cultivation field exhibited extremely high soil EC values, low seed germination rates, and severe soil compaction. However, as shown in FIG. 9B, three months after applying the functional microbiome, the soil EC value was significantly reduced, the seed germination rate increased, and soil compaction was markedly improved. Additionally, plant yield increased by approximately 60%. These results demonstrate that the functional microbiome selected using the method of the present invention exhibits effective soil restoration capability.

In summary, the selecting method provided in the present invention utilizes local samples for microbial selection. The microorganisms originally present in the local sample already possess adaptive capabilities suited to the local environment, and the selected and domesticated functional microbiome does not disrupt the native microbial balance by introducing foreign species. Furthermore, the present invention utilizes a composite microbiome instead of a single microbial strain, thereby improving environmental adaptability, enhancing survival rates after introduction into the restoration site, and significantly increasing the success rate of environmental restoration. Additionally, experimental results confirm that the functional microbiome selected according to the present invention effectively enhances seed germination and achieves substantial improvements in soil fertility. Therefore, the present invention has broad application value in environmental restoration and agricultural development.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A method for selecting functional microbiome, comprising:
providing a local soil sample;
performing a pretreatment on the local soil sample to obtain a pretreated soil sample, and
filtering the pretreated soil sample to obtain a first microbial suspension;
culturing the first microbial suspension using a culture formulation to obtain a second microbial suspension; and
plating the second microbial suspension to determine the content of *Bacillus subtilis* therein, wherein the second microbial suspension is designated as functional microbiome when the content of *Bacillus subtilis* exceeds 5% of the total microbial population.

2. The method of claim 1, wherein the local soil sample comprises earthworms and/or metabolites derived from earthworms.

3. The method of claim 1, wherein the pretreatment comprises a heat treatment and/or an extractant treatment.

4. The method of claim 3, wherein the heat treatment comprises heating the local soil sample in an extractant at a temperature of 60-90°C for 30-60 minutes.

5. The method of claim 3, wherein the extractant treatment comprises adding an extractant to the local soil sample to perform extraction.

6. The method of claim 4 or claim 5, wherein the extractant comprises at least one selected from the group consisting of water, physiological saline, culture medium, a bacitracin-containing culture medium, a monoculture supernatant, a saline suspension, and a metal ion suspension.

7. The method of claim 1, wherein the culture formulation comprises at least one selected from the group consisting of glucose, glycerol, molasses, starch, arabinose, fructose, galactose, lactose, maltose, mannose, sucrose, peanut meal, soybean meal, corn gluten meal, beef extract, peptone, yeast powder, fish meal, urea, spent fungal biomass, fermented residue with ammonium salts, nitrate salts, ammonia water, yeast extract, sodium chloride, magnesium chloride, sodium bicarbonate, monopotassium phosphate, dipotassium phosphate, ammonium sulfate, ammonium chloride, sodium citrate, sodium malate, sodium carbonate, and potassium carbonate.

8. A method for soil restoration, comprising:
providing the functional microbiome selected according to the method of claim 1;
subjecting the functional microbiome to microbial domestication to obtain domesticated functional microbiome, wherein the microbial domestication comprises culturing the functional microbiome in a bioreactor and regulating microenvironmental parameters in the bioreactor to simulate the environment of the local soil sample; and
introducing the domesticated functional microbiome into the local soil.

9. The method of claim 8, wherein the microenvironmental parameters comprise nutrient composition, nutrient concentration, pH, temperature, humidity, mechanical force, and a growth medium.

10. The method of claim 8, wherein the growth medium comprises wood chips, rice husks, and soil.
